# FASCICULE DE BREVET EUROPEEN

(11) **EP 0 505 554 B1**
(45) Date de publication et mention de la délivrance du brevet: **03.05.1995**
(21) Numéro de dépôt: 91919787.1
(22) Date de dépôt: 15.10.1991
(51) Int. Cl.: A61B 5/103, G01N 1/00

(54) **DISPOSITIF PERMETTANT LE DOSAGE D'AU MOINS UN COMPOSE DIRECTEMENT SUR LA PEAU ET PROCEDE DE DOSAGE CORRESPONDANT**
GERÄT, DAS DIREKT AUF DIE HAUT DIE BESTIMMUNG DER MENGE MINDESTENS EINES SUBSTRATES ERMÖGLICHT UND ENTSPRECHENDES VERFAHREN
DEVICE FOR ANALYZING AT LEAST ONE COMPOUND DIRECTLY ON THE SKIN AND CORRESPONDING METHOD OF ANALYSIS

(30) Priorité: 16.10.1990 FR 9012731
(43) Date de publication de la demande: 30.09.1992
(73) Titulaire: L'OREAL, 75008 Paris (FR)
(72) Inventeur: BAZIN, Roland, F-94400 Vitry-sur-Seine (FR); BERNARD, Dominique, F-60200 Compiègne (FR); OBADIA, Gérard Résidence Solimar, F-06200 Nice (FR); MARCOTTE, Louis, F-94150 Chevilly-Larue (FR)
(74) Mandataire: Peuscet, Jacques
(86) Numéro de dépôt international: FR9100801
(87) Numéro de publication internationale: WO9206635

(56) Documents cités:
- EP-A- 0 340 774
- DD-A- 246 313
- FR-A- 2 640 643

## Description

La présente invention concerne un dispositif permettant le dosage d'un ou plusieurs composé(s) directement sur la peau et un procédé de mesure correspondant.

Le dosage direct sur la peau de composés endogènes et exogènes présente un grand intérêt en médecine, car il permet de diagnostiquer certaines maladies de la peau ; en outre, dans la recherche en médecine et en cosmétologie, il pourrait permettre soit de suivre certains processus biologiques de la peau, tels que le métabolisme énergétique de la peau ou la relation entre les amino-acides libres présents sur la peau et la kératinisation, soit d'évaluer l'action de composés pharmaceutiques et/ou cosmétiques sur la peau.

Il est connu, par exemple, que l'application d'un lactate sur la peau a une action hydratante en raison de ses propriétés hygroscopiques (Van Scott et al., J. Am. Acad. Dermatol., 1984, 11, 869 ; et Takahashi et al., J. Soc. Cosmet. Chem., 1985, 36 -177). On sait, par ailleurs, qu'une augmentation du taux de lactate est un signe d'hyperprolifération cellulaire dans les couches profondes de la peau. Dans ces conditions, on comprend que des dosages du lactate présent sur la peau permettent d'étudier son rôle dans l'hydratation de la peau ou de faire le diagnostic d'une hyperprolifération cellulaire.

Une méthode de dosage connue consiste à mettre l'échantillon contenant le composé à doser en contact avec une enzyme, qui catalyse une transformation du composé à doser, et à mesurer un phénomène provoqué par la transformation. Par exemple, dans le cas des lactates, en présence de lactate oxydase (E.C.1.1.3.2) de Pediococcus Sp., la réaction suivante se produit en présence d'oxygène :

L-lactate + O₂ → pyruvate + H₂O₂

Pour doser le lactate, on peut donc le mettre en présence de lactate oxydase et mesurer soit la consommation d'oxygène, soit la production d'eau oxygénée. Il est, en particulier, connu d'utiliser pour effectuer ce dosage, une électrode enzymatique comportant, d'une part, un dispositif électrochimique de détection ampèremétrique à potentiel imposé du phénomène provoqué par la transformation enzymatique (par exemple, mesure de la pression partielle d'oxygène) et, d'autre part, une membrane enzymatique appliquée à l'extrémité sensible de l'électrode.

L'application directe de la membrane de l'électrode enzymatique sur la peau s'est révélée impossible et, dans FR-A 2640 643, la demanderesse a proposé d'associer à l'électrode une cellule de mise en solution du composé à doser, dite cellule d'échanges cutanés, dans laquelle on peut faire circuler un milieu liquide approprié tel qu'un tampon, la cellule comportant une zone de paroi constituée par la membrane enzymatique et une ouverture qui peut être obturée par une zone de peau lors de la mesure. Dans ce procédé, on dissout donc le composé à doser par circulation d'un liquide de dissolution sur la peau et on mesure la teneur en composé dans la solution obtenue.

Le biocapteur décrit ci-dessus est avantageusement utilisé lorsque l'étude ou le diagnostic effectué nécessite le dosage d'un seul composé en présence d'une enzyme donnée avec la mesure d'un seul phénomène provoqué par la transformation enzymatique du composé à doser. Mais ce biocapteur est difficilement utilisable lorsque l'étude ou le diagnostic nécessite la mesure simultanée ou successive de plusieurs composés. Le changement de la membrane étant assez malaisé, on est amené à utiliser simultanément ou successivement plusieurs biocapteurs comportant chacun une cellule de mise en solution du composé à doser. Les mesures obtenues sont donc moins fiables et moins comparables, car ou bien elles ne proviennent pas d'une même zone de peau, ou bien elles proviennent d'une zone de peau qui a déjà été balayée par le liquide de dissolution.

La présente demande a pour but de fournir un dispositif de mesure qui permette d'effectuer simultanément le dosage de plusieurs composés différents provenant d'une même zone de peau et qui a, également, l'avantage de rendre possible la réalisation de séquences de mesure dans un processus de mesure automatique.

La présente invention a pour objet un dispositif permettant le dosage direct sur la peau d'un ou plusieurs composé(s) comportant une cellule de mise en solution du composé à doser à l'aide d'un liquide de dissolution circulant sur la peau dans la cellule ; et, au moins une électrode enzymatique, caractérisé par le fait qu'il comprend :
- un réservoir pour le liquide de dissolution,
- une cellule de mise en solution alimentée par ledit réservoir,
- au moins une électrode enzymatique, dont la membrane est en contact avec le liquide de dissolution provenant de la cellule de mise en solution et
- au moins une pompe permettant de faire circuler le liquide de dissolution, de la cellule de mise en solution vers l'(les) électrode(s) enzymatique(s) avec un retour au réservoir pour constituer un circuit fermé.

Dans ce dispositif, la cellule de mise en solution et l'(les) électrode(s) est (sont) séparée(s), ce qui permet une grande souplesse dans les mesures. Dans ces conditions la cellule de mise en circulation peut être constituée par un dispositif simple, maniable et complètement isolé électriquement, assurant une complète sécurité pour l'individu sur lequel le (ou les) composé (s) sont testés.

Par ailleurs dans le dispositif selon l'invention le liquide de dissolution circule en circuit fermé pendant le dosage. On peut ainsi concentrer dans le liquide de dissolution la molécule que l'on veut doser grâce au dit recyclage et obtenir des concentrations détectables par la plupart des techniques de dosage. Le recyclage sur la peau permet de récupérer au mieux le composé à doser même si ce composé est difficilement solubilisable. Par ailleurs la dissolution de celui-ci peut être suivie en direct dans le circuit, en mesurant les variations de concentrations. On peut donc ainsi évaluer aussi bien les phénomènes de disponibilité que la production du composé que l'on teste.

Une cuve de stockage du liquide de dissolution est avantageusement reliée au réservoir.

Le réservoir est, de préférence, thermostaté de façon à maintenir à une température constante, de préférence proche de la température cutanée moyenne (3O°C), le liquide de dissolution. Ce thermostatage se fait avantageusement, par une double enveloppe dans laquelle circule un fluide échangeur, mais il peut également être fait à l'aide d'une résistance électrique. Le réservoir est muni de deux conduits, l'un d'entrée et l'autre de sortie, permettant la circulation en cycles fermés du liquide de dissolution lors du fonctionnement ; il peut aussi comporter un conduit de soutirage permettant de vidanger le liquide de dissolution après le dosage et un conduit d'alimentation relié à la cuve de stockage permettant le remplissage du réservoir avant dosage. Le réservoir comporte à partir du niveau des conduits permettant la circulation du liquide de dissolution, un volume suffisant pour que le liquide de dissolution puisse remplir la totalité du circuit de dosage. Comme expliqué plus en détail ci-après, le réservoir peut servir de support aux électrodes.

Le liquide de dissolution est, de préférence, constitué par une solution tampon, dont la nature et le pH peuvent varier en fonction du (ou des) composé (s) à doser. Dans le cas où on utilise une électrode de platine, la solution tampon est, par exemple, une solution de phosphate.

La cellule de mise en solution du composé à doser présente une zone de paroi ouverte disposée de façon telle que la peau puisse servir de paroi de fermeture lorsque cette cellule est appliquée sur la peau. Elle est, par exemple, constituée par une plaquette de matière plastique, rigide ou souple, plate ou bombée, sur une des faces de laquelle est ménagé en creux un compartiment ouvert. Ce compartiment a, avantageusement, la forme d'une conduite enroulée en spirale ou repliée en zig-zag comme décrit dans la demande de brevet français FR-A-2 667 778. La cellule de mise en solution peut être fixée sur un dispositif, par exemple un brassard, qui garantit un appui uniforme et/ou une pression constante contrôlable sur la peau.

L' (les) électrode(s) enzymatique(s) est (sont), de préférence, associée(s) à au moins une électrode de référence, qui a la même structure que l' (les) électrode(s) enzymatique(s) à laquelle (auxquelles) elle (s) est (sont) associée(s) à l'exception du fait que la membrane ne contient pas d'enzyme. La présence d'une électrode de référence permet d'éliminer les interférences qui seraient dues à la présence éventuelle d'espèces électroactives sur la peau.

Les électrodes sont, de préférence, associées à un ampèremètre d'alimentation à potentiel imposé maintenant une tension déterminée constante et le signal de mesure est donc une intensité repérée, en général, en nanoampères (nA).

On peut utiliser des électrodes enzymatiques de types différents de façon à doser simultanément sur la peau plusieurs composés. On peut aussi utiliser plusieurs électrodes enzymatiques de même type, de façon à obtenir simultanément plusieurs mesures pour un même composé, ce qui permet de corriger les écarts qui seraient dus à l'électrode.

Le type d'électrode utilisé dépend du composé à doser et du procédé de dosage utilisé.

Par exemple dans le cas où le composé à doser est un L-lactate, on peut utiliser une des nombreuses électrodes à enzyme utilisant les systèmes enzymatiques ci-après :
a) enzymes catalysant la réaction suivante :

   L-lactate + O₂ → acétate + CO₂ + H₂O

   associées à un dispositif électrochimique de détection ampèremétrique à potentiel imposé de la pression partielle d'oxygène, l'enzyme pouvant être la lactate déshydrogénase (E.C.1.1.1.27), le cytochrome b2 (E.C.1.1.2.3) ou la lactate 2-monooxygénase (E.C.1.13.12.4) de Mycobacterium Smegmatis.
b) enzymes catalysant la réaction suivante :

   L-lactate + O₂ → pyruvate + H₂O₂

   associées à un dispositif électrochimique de détection ampèremétrique à potentiel imposé de la pression partielle d'oxygène ou de la teneur en H₂O₂, l'enzyme étant en particulier la lactate oxydase (E.C.1.1.3.2)

On utilise, par exemple, une électrode de Clark, qui est, en particulier, décrite dans Anal. Chem. Acta, 1984, 157, page 45.

Dans le cas où le composé à doser est un amino-acide, on utilise avantageusement une électrode de platine, dans la membrane de laquelle est immobilisée la L-amino-acide oxydase (E.C.1.4.3.2) qui catalyse la réaction suivante :

R-CH(NH₂)COOH + O₂ + H₂O → R-COCOOH + NH₃ + H₂O₂

en association avec un dispositif électrochimique de détection ampèremétrique à potentiel imposé de H₂O₂. Dans ce dispositif, l'eau oxygénée libère de l'oxygène par la réaction électrochimique

H₂O₂ → O₂ + 2H⁺ + 2e⁻

à l'interface de l'anode de platine et de la membrane ; la cathode est en argent chloruré.

On peut également, selon l'invention, doser d'autres composés à l'aide d'électrodes de platine en immobilisant dans la membrane d'autres enzymes : notamment, la lactate oxydase (E.C.1.1.3.2) pour doser le L-lactate, la cholestérol oxydase (E.C.1.1.3.6) pour doser le cholestérol, une phospholipase avec la choline oxydase pour doser les phospholipides, par exemple la phospholipase D (E.C.3.1.4.4) avec la choline oxydase (E.C.1.1.3.17) pour doser la lécithine.

Les électrodes sont, de préférence, fixées sur des supports. De préférence, on fixe sur un même support plusieurs électrodes enzymatiques du même type ainsi que l'électrode de référence correspondante ; par conséquent si l'on utilise plusieurs types d'électrodes, on dispose avantageusement sur le circuit plusieurs supports différents, chaque support contenant un type déterminé d'électrode(s) enzymatique(s) et, éventuellement, l'électrode de référence correspondante.

Selon un mode de réalisation préféré le réservoir et le support d'électrode sont combinés, le réservoir comportant des éléments de support pour les électrodes. Dans ce but, on insère dans la paroi du réservoir au moins un embout, ou on ménage au moins une alvéole, s'ouvrant à l'intérieur du réservoir et ayant une forme ou des dimensions telles que l'on puisse introduire de façon étanche l'électrode, la membrane la première ; la membrane enzymatique de l'électrode est alors en contact avec le liquide de dissolution contenu dans le réservoir. De préférence, on dispose radialement quatre embouts, ou ménage quatre alvéoles, disposées en croix. Ce mode de réalisation a l'avantage de permettre d'effectuer l'étalonnage directement dans le réservoir avant le remplissage du circuit de mesure. On évite ainsi d'avoir à déconnecter la cellule d'échange, à remplir et à rincer le circuit. De plus, on évite d'éventuelles variations de pression au niveau de la membrane des électrodes qui risqueraient d'engendrer une instabilité de la ligne de base du signal.

Le liquide de dissolution peut être maintenu en circulation dans le circuit à l'aide d'une seule pompe, qui est alors, de préférence, disposée en aval de l' (des) électrode(s). On peut cependant utiliser, pour assurer la circulation du liquide de dissolution, deux pompes, auquel cas l'une est avantageusement disposée en amont de la cellule de mise en solution et l'autre en aval. La (les) pompe(s) est (sont), de préférence, une (des) pompe(s) péristaltique(s). Lorsque la circulation du liquide de dissolution est assurée par deux pompes, la capacité d'aspiration de la pompe située en aval de la cellule est, de préférence, supérieure à la capacité de refoulement de la pompe située en amont, de façon à créer dans la cellule de mise en solution une légère dépression et garantir une meilleure application, donc une meilleure étanchéité, de la cellule sur la peau.

Pour automatiser le processus de dosage, le circuit peut aussi comprendre plusieurs autres pompes, de préférence des pompes péristaltiques, disposées en particulier sur le conduit d'alimentation du réservoir et sur le conduit de vidange du réservoir ; le circuit est alors, de préférence, associé à une unité d'acquisition des informations fournies par la (ou les) électrode(s) et de commande des pompes de circulation du liquide de dissolution.

La présente demande a également pour objet un procédé de dosage direct sur la peau d'au moins un composé utilisant le dispositif décrit ci-dessus, caractérisé par les opérations suivantes :
- on remplit le réservoir avec le liquide de dissolution ;
- on applique la cellule de mise en solution du (des) composé (s) à doser sur la zone de peau choisie ;
- on met en fonctionnement la (les) pompe(s) de circulation du liquide de dissolution et on met sous tension constante l' (les) électrode(s) enzymatique(s) et, éventuellement, de référence ;
- on maintient la circulation du liquide de dissolution et, simultanément, on repère l'intensité à la sortie de la (ou des) électrode(s) enzymatique(s) et éventuellement de référence, pour définir la quantité du (ou des) composé(s) dosé(s) ;
- on rince le circuit de circulation à l'aide de liquide de dissolution pur ; et
- on vidange ledit circuit.

Les différentes opérations sont, de préférence, commandées par le système informatique.

Le remplissage du réservoir est, de préférence, effectué en faisant fonctionner une pompe située sur le conduit reliant le réservoir à la cuve de stockage pendant un temps déterminé, fonction du volume du réservoir et du débit de la pompe. La fin du remplissage pourrait également être déterminée par un capteur de niveau.

Il a été constaté que, dans le cas où la concentration du produit sur la peau ne varie pas, la quantité de produit dosée croît en fonction du temps de dissolution pour atteindre un palier. On arrête donc la mesure lorsque la quantité de produit détectée dans le liquide de circulation ne varie pratiquement plus en fonction du temps ; en d'autres termes, la valeur du signal fourni par l' (les) électrode(s) est prise en compte comme valeur de mesure lorsque la variation de cette valeur en fonction du temps devient inférieure à un seuil prédéfini.

On peut aussi mesurer une quantité de produit dont la concentration sur la peau varie au cours du temps, par exemple, un composé constituant de la sueur. Dans ce cas, on prend comme valeur de mesure la pente d'évolution du signal en fonction du temps, car, dans le cas d'un phénomène dynamique, il n'y a pas de stabilisation du signal. On peut ainsi suivre cinétiquement un phénomène tel que la sudation naturelle ou provoquée.

Le temps de rinçage est, de préférence fonction de la concentration maximale du composé dosé sur la peau dans le liquide de dissolution.

Pour mieux faire comprendre l'objet de l'invention, on va en décrire ci-après, à titre purement illustratif et non limitatif et en se référant au dessin annexé, un mode de réalisation et le procédé de fonctionnement correspondant.

Sur ce dessin :
- la figure 1 représente schématiquement un dispositif selon l'invention ;
- la figure 2 représente, en fonction du temps, la courbe donnant la différence I de l'intensité mesurée à la sortie d'une électrode enzymatique et de l'intensité mesurée à la sortie de l'électrode de référence correspondante et le diagramme de commande des différentes phases du procédé ;
- la figure 3 représente plus en détail le réservoir du dispositif de la figure 1 ;
- les figures 4 et 5 représentent une cellule de mise en solution, respectivement vue de dessus et vue en coupe selon V-V de la figure 4 ;
- la figure 6 représente, en perspective, un support pour les électrodes ;
- la figure 7 représente une coupe selon VII-VII de la figure 6 et une électrode prête à être introduite dans son support ;
- les figures 8 et 9 représentent une autre forme de support d'électrodes, la figure 8 étant une vue selon VIII-VIII de la figure 9 et la figure 9 une vue selon IX-IX de la figure 8 ;
- la figure 10 représente en coupe longitudinale un réservoir muni d'éléments support d'électrodes ;
- la figure 11 représente en coupe transversale selon XI-XI de la figure 10 du même réservoir ;
- la figure 12 représente en coupe longitudinale selon XII-XII de la figure 1O dudit réservoir ;
- la figure 13 est une vue de dessus dudit réservoir.

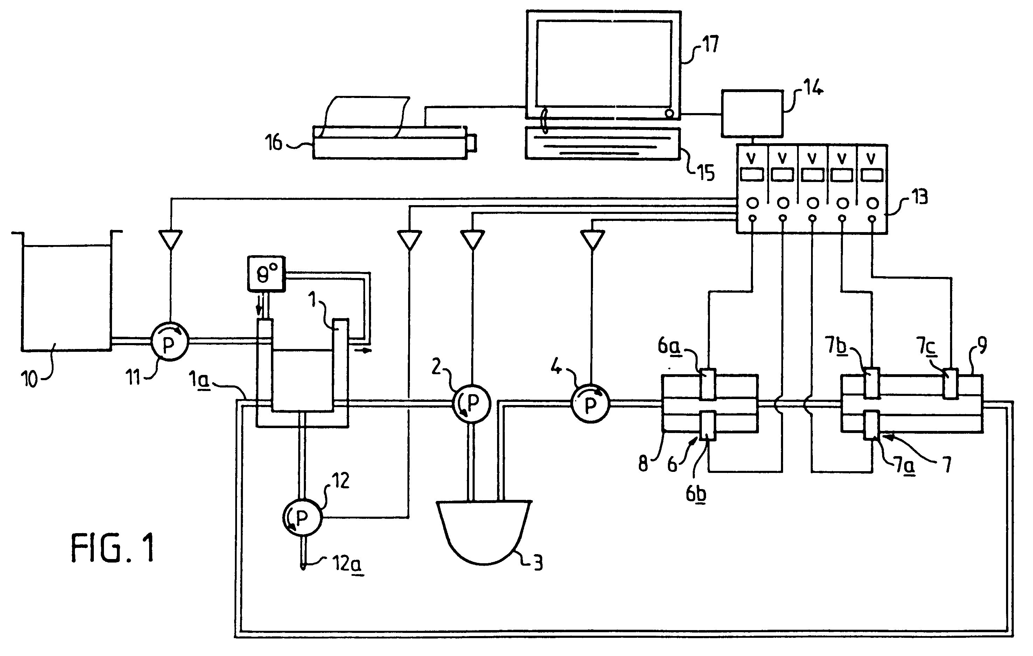

En se référant à la figure 1, on voit que le dispositif de dosage selon l'invention comprend un réservoir 1 relié par l'intermédiaire d'une pompe péristaltique 2 à une cellule 3 de mise en solution. La cellule 3 est reliée par l'intermédiaire d'une pompe péristaltique 4 à deux groupes d'électrodes enzymatiques 6 et 7 fixées en série respectivement sur des supports 8 et 9.

Le premier groupe d'électrodes fixé sur le support comporte une électrode de mesure 6a, qui est une électrode de Clark et une électrode de référence 6b qui est identique à l'électrode 6a, sauf que la membrane n'a pas été imprégnée d'enzyme. Le liquide de dissolution traverse le support 8 et entre en contact avec les membranes des différentes électrodes.

Le second groupe d'électrodes enzymatiques comporte, selon le mode de réalisation représenté, trois électrodes 7a, 7b et 7c. Les électrodes 7a et 7b sont des électrodes enzymatiques de platine permettant de doser les amino-acides et l'électrode 7c est une électrode de référence identique aux électrodes 7a, 7b et 7c sauf que la membrane n'est pas imprégnée d'enzyme. Le liquide de dissolution sortant du support 9, où il est mis en contact avec les électrodes de platine, est recyclé dans le réservoir 1 par le retour 1a. Le liquide de dissolution circule donc en circuit fermé. Le dispositif est complété par une cuve de stockage 10 du liquide de dissolution reliée par une pompe 11 au réservoir 1. Le réservoir 1 est relié à une vidange 12a par l'intermédiaire d'une pompe 12.

Les différentes électrodes enzymatiques et de référence sont reliées à une source électrique régulée fournissant une tension constante et à un appareil 13 de mesure de l'intensité du courant et pour chaque électrode enzymatique, le signal pris en compte est la différence ΔI, entre l'intensité mesurée à la sortie de l'électrode enzymatique et l'intensité mesurée à la sortie de l'électrode de référence. Le signal analogique ΔI est transformé en données numériques par un convertisseur analogique digital 14 et le signal numérique ainsi obtenu est transmis à un ordinateur 15 qui, à partir d'une table d'étalonnage, le transforme en une valeur de la concentration en composé dosé, valeur qui peut être visualisée sur une imprimante 16 ou un écran 17 branchés sur l'ordinateur.

Les différentes pompes 2, 4, 11 et 12 sont des pompes péristaltiques. Les pompes 2 et 4 assurent la circulation du liquide de dissolution ; la pompe 4 a une capacité d'aspiration supérieure à la capacité de refoulement de la pompe 2 : si les deux pompes sont identiques, le rotor de la pompe 4 peut par exemple tourner plus vite que celui de la pompe 2. De la sorte, on crée dans la cellule 3 une légère dépression, qui applique la cellule 3 sur la peau et permet d'améliorer son étanchéité.

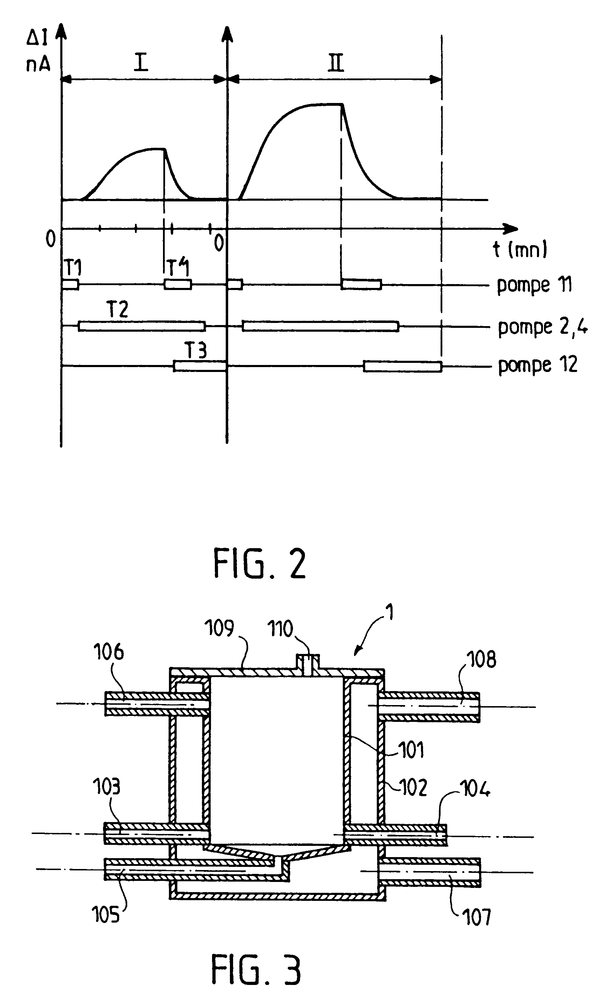

La figure 3 représente plus en détail un mode de réalisation du réservoir 1. Il se compose d'une cuve 101 cylindroconique, entourée d'une chemise cylindrique 102. A la partie inférieure de la partie cylindrique de la cuve se trouvent deux conduits 103 et 104 permettant la circulation du liquide de dissolution, le conduit 103, relié au retour 1a, permettant l'introduction du liquide dans le réservoir et le conduit 104 son soutirage. A la pointe de la partie conique de la cuve 101 est disposé un conduit 105 de vidange de la cuve 101 ; ce conduit 105 est partiellement contenu dans la chemise 102. Un conduit d'alimentation en liquide 1O6 est disposé à la partie supérieure de la cuve 101. La chemise 102 est pourvue de deux conduits 1O7 et 1O8 d'alimentation et de soutirage d'un fluide échangeur de chaleur. La cuve 1O1 est fermée par un couvercle 1O9 muni d'un conduit 11O permettant l'introduction d'une solution étalon de lactate et/ou d'amino-acide pour l'étalonnage préalable.

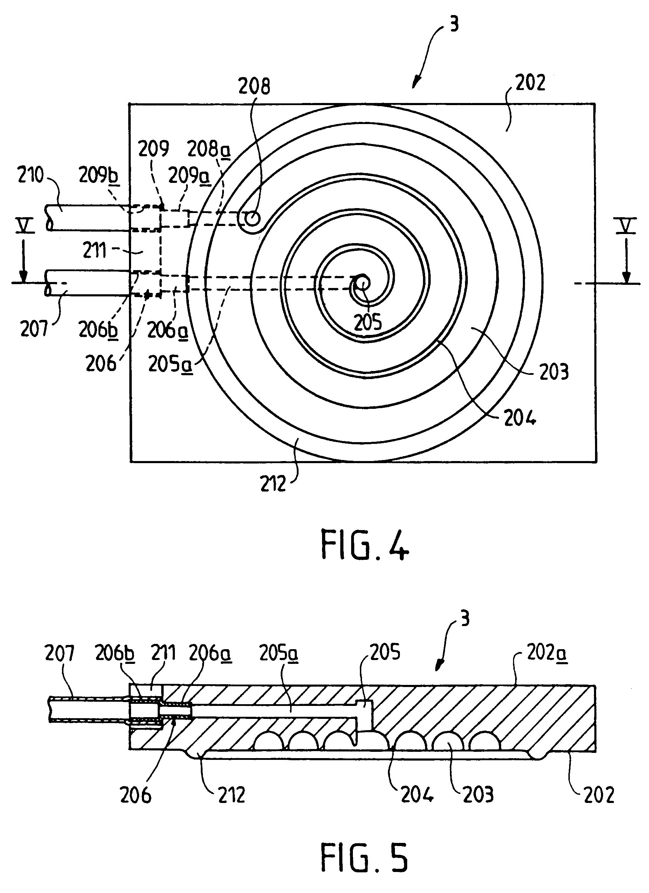

Les figures 4 et 5 montrent plus en détail un mode de réalisation préféré de la cellule 3 de dissolution également appelée cellule d'échanges cutanés. Elle se présente sous la forme d'une plaquette plane rectangulaire 2O2 réalisée en matière plastique transparente. Sur une face de cette plaquette 2O2 est ménagée une conduite ouverte 2O3 enroulée en forme de spirale. Chacune des spires de la conduite 2O3 est séparée de la suivante par une mince cloison 2O4. La conduite 2O3 en spirale a une section constante sur toute sa longueur. Au centre de la spirale s'ouvre un conduit 2O5 qui est perpendiculaire aux faces de la plaquette 2O2 et qui est relié à un conduit 2O5a ménagé dans l'épaisseur de la plaquette 2O2, parallèlement aux faces de ladite plaquette, entre la conduite 2O3 en spirale et la face 2O2a de la plaquette 2O2 opposée à celle où s'ouvre la conduite 2O3 en spirale. Ce conduit 2O5a est relié par un embout métallique 2O6, inséré dans la matière plastique, à un tube souple 2O7 en matière plastique. A l'extrémité extérieure de la spirale de la conduite 2O3 s'ouvre également un conduit 2O8 perpendiculaire aux faces de la plaquette 2O2 qui est relié à un conduit 2O8a ménagé dans l'épaisseur de la plaquette 2O2, parallèlement au conduit 2O5a. Ce conduit 2O8a est, comme le conduit 2O5a, relié par un embout 2O9, identique à l'embout 2O6, à un tuyau souple 21O. Les conduits 2O5, 2O5a, 2O8, 2O8a, 2O7 et 21O ont une section droite interne ayant la même surface que la section droite interne de la conduite 2O3 en spirale. Les embouts métalliques 2O6 et 2O9 sont cylindriques et constitués de deux parties de diamètres différents. Les parties de plus petits diamètres 2O6a et 2O9a sont insérées dans l'épaisseur de la plaquette 2O2. Les parties de plus grands diamètres 2O6b et 2O9b sont disposées, à l'extérieur de la plaquette 2O2, dans une échancrure parallélépipédique 211 ménagée sur le bord de ladite plaquette 2O2. Les tubes souples 2O7 et 21O sont enfoncés respectivement sur les parties 2O6b et 2O9b des embouts 2O6 et 2O9. Un bourrelet périphérique 212 entoure la conduite en spirale 2O3 et améliore l'étanchéité.

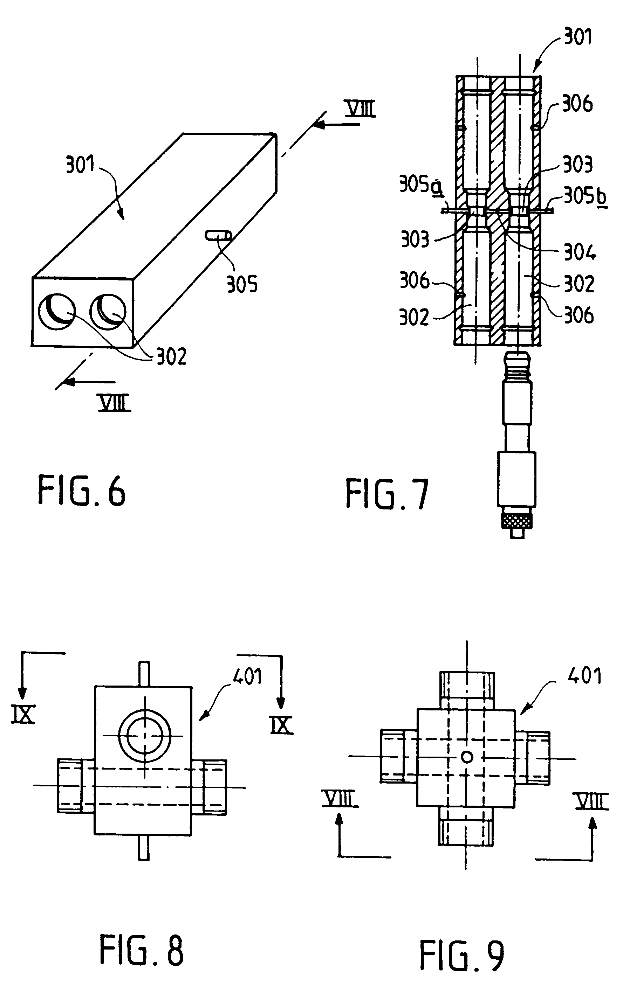

Les figures 6 à 9 représentent plus en détail des supports 6 et 7 utilisables pour les électrodes enzymatiques.

Les figures 6 et 7 représentent une première variante de support permettant de fixer quatre électrodes enzymatiques. Ce support est constitué par un bloc parallélépipédique 3O1 en matière plastique moulée. Dans ce bloc sont ménagées quatre alvéoles 3O2 ayant des dimensions et une forme adaptées aux électrodes que l'on désire y introduire. Comme représenté sur la figure 7, les alvéoles 3O2 sont disposées deux par deux parallèlement au grand axe du bloc 3O1, de façon que les électrodes puissent être introduites deux par deux dans le bloc 3O1 par chacune des faces les plus éloignées du bloc 3O1, la partie de l'électrode portant la membrane enzymatique étant introduite en premier. Les alvéoles 3O2 débouchent dans deux chambres 3O3 communes à deux alvéoles opposées ; ces chambres sont reliées entre elles par un conduit 3O4 ménagé à l'intérieur du bloc 3O1 et sont reliées à l'extérieur par deux tuyaux 3O5a et 3O5b insérés dans le bloc 3O1. Les tuyaux 3O5a et 3O5b et le conduit 3O4 sont alignés et parallèles au petit axe du bloc 3O1. Des tiges 3O6 permettent de bloquer le capteur à oxygène à l'intérieur du support.

Les figures 8 et 9 représentent un support 4O1 permettant d'utiliser simultanément quatre électrodes disposées en croix, les deux branches de la croix étant décalées l'une par rapport à l'autre.

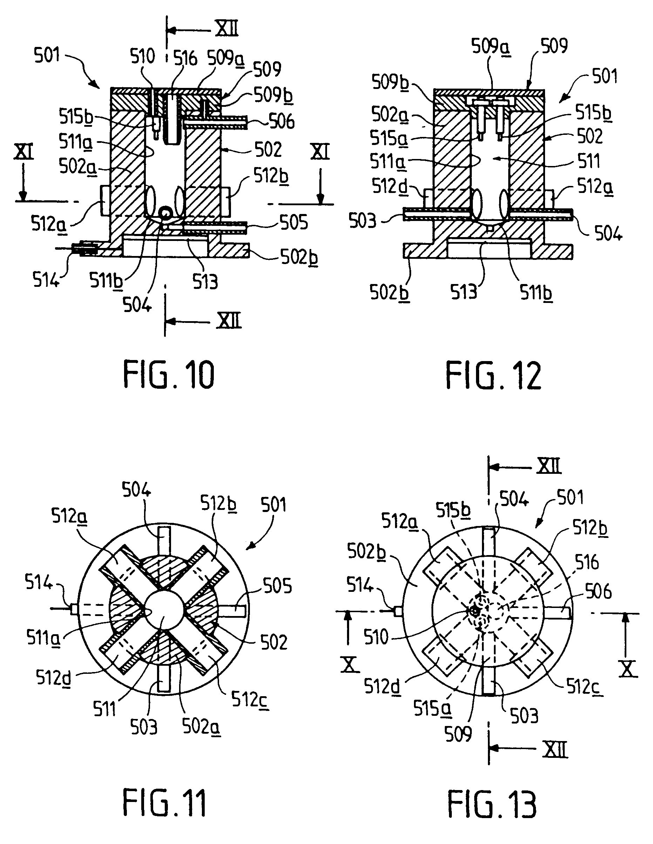

Les figures 1O à 13 représentent un second mode de réalisation du réservoir, ce réservoir contenant des éléments supports pour des électrodes.

Le réservoir désigné dans son ensemble 5O1 est constitué par une enveloppe 5O2 en substance calorifuge comportant une partie cylindrique extérieurement 5O2a reposant sur un disque 5O2b de plus grand diamètre. A l'intérieur de l'enveloppe est ménagée une cuve 511 de forme cylindroconique destinée à contenir le liquide de dissolution. A la partie inférieure de la partie cylindrique 511a de la cuve 511 se trouvent deux conduits 5O3 et 5O4, permettant la circulation du liquide de dissolution, qui sont diamètralement opposés. Le conduit 5O3 est relié au retour 1a du circuit et le conduit 5O4 permet le soutirage. A la pointe de la partie conique 511b de la cuve 511 est disposé un conduit 5O5 de vidange de la cuve 511. Ce conduit comporte une partie parallèle à l'axe du réservoir et une partie perpendiculaire à cet axe s'étendant radialement vers l'extérieur de la partie 5O2a de l'enveloppe 5O2. La partie supérieure de la cuve 511 est munie d'un conduit d'alimentation 5O6 en liquide de dissolution neuf provenant d'une cuve de stockage. Au-dessus du plan contenant l'axe des deux conduits 5O3 et 5O4, sont disposés, selon le mode de réalisation représenté, quatre embouts 512a, 512b, 512c, 512d, dont la paroi intérieure est cylindrique ; ils sont destinés à servir de support pour quatre électrodes, par exemple trois électrodes de mesure et une électrode de référence. Ces supports dont l'axe est disposé dans un même plan, sont diamétralement opposés deux à deux et forment une croix. Ces embouts 512a, 512b, 512c, 512d font saillie à l'extérieur de l'enveloppe 5O2 par une partie épaissie et s'ouvrent au ras de la paroi 511a de la cuve 511. Dans la partie en forme de disque 5O2b de l'enveloppe 5O2 est ménagée une échancrure de forme cylindrique dans laquelle est logée une résistance chauffante 513. Une prise de courant 514 pour alimenter la résistance 513 est prévue à la périphérie du disque 5O2b. Le réservoir 5O1 est fermé par un couvercle 5O9 constitué par un chapeau 5O9a et par une rondelle 5O9b. La rondelle 5O9b et le chapeau 5O9a sont traversés par un conduit 51O permettant l'introduction d'une solution étalon, par exemple de lactate ou d'amino-acide. Sous la rondelle 5O9b sont fixés des détecteurs de niveau 515a et 515b qui traversent par des échancrures appropriées le chapeau 5O9a. Sous la rondelle 5O9b est également fixé un capteur de température 516 qui traverse le chapeau par une ouverture appropriée. La cuve 511 a un volume tel qu'elle puisse contenir suffisamment de liquide pour remplir le circuit et pour maintenir en contact avec le liquide de dissolution la membrane d'électrodes introduite dans les embouts 512a, 512b, 512c, 512d, le niveau du liquide après remplissage de la cuve 511 étant contrôlé par les détecteurs de niveau 515a et 515b. Avec ce type de réservoir, il est possible d'étalonner les électrodes fixées sur ce réservoir, sans qu'il soit nécessaire de remplir le circuit. On évite ainsi de remplir, puis rincer le circuit.

Le dispositif fonctionne de la façon décrite ci-après. On commence par enregistrer dans l'ordinateur 15 tous les paramètres du dispositif tels que la surface de la cellule cutanée, le volume de liquide de dissolution contenu dans l'ensemble du circuit, ainsi que les informations propres à la mesure telles que l'individu et le site cutané. On fait ensuite au moins une mesure avec une solution étalon ayant une concentration déterminée en produit à doser. La variation de l'intensité en fonction de la concentration étant linéaire dans la gamme de concentration choisie, il suffit généralement de mesurer un point. On introduit le résultat de la mesure dans l'ordinateur. On demande à l'ordinateur de commander la mise en route de la pompe 11 de façon à introduire dans le réservoir 1 une quantité de liquide de dissolution suffisante pour remplir l'ensemble du circuit, c'est-à-dire l'ensemble des conduits formant le circuit, la cellule 3, les chambres des supports 8 et 9 et la partie du réservoir située au-dessous des conduits d'alimentation et de soutirage. Puis on commande l'arrêt de la pompe 11 au bout d'un temps déterminé au préalable, qui est celui nécessaire, avec un débit de liquide déterminé, pour remplir le réservoir 1. Simultanément, on met en route la circulation de liquide de thermostatage du réservoir 1 et on laisse la température du liquide de dissolution s'équilibrer à environ 3O°C.

On applique ensuite la cellule 3 de mise en solution sur le site de mesure, par exemple sur le bras d'un individu, à l'aide d'un brassard de maintien. On relie la cellule 3 au circuit par les tuyaux souples 2O7 et 21O en un point du circuit situé entre le réservoir 1 et les électrodes enzymatiques 6a, 6b.

Dans un premier procédé de mesure pour mesurer la concentration en un produit, l'ordinateur commande alors la mise en fonction des pompes péristaltiques 2 et 4 et la mise sous tension constante des électrodes enzymatiques de mesure et des électrodes de référence ; on mesure en nanoampères le signal, qui est constitué par la différence d'intensité ΔI à la sortie d'une électrode de mesure et de l'électrode de référence correspondante. L'ordinateur suit la variation de cette différence d'intensité et calcule la pente de la courbe représentant ΔI en fonction du temps. Lorsque la pente de la courbe de ΔI est sensiblement nulle, c'est-à-dire inférieure à un seuil prédéfini, l'ordinateur calcule, à partir du signal en nanoampères, les résultats en nanomoles par cm² de surface cutanée, par comparaison avec la courbe d'étalonnage établie au préalable.

Puis l'ordinateur commande le rinçage du circuit en mettant en route la pompe 11 pour alimenter le réservoir 1 en liquide de dissolution neuf, puis la pompe 12 pour le vidanger ; les pompes 2 et 4 continuent à fonctionner. Ensuite, l'ordinateur commande l'arrêt de la pompe 11, puis des pompes 2 et 4 tout en maintenant la pompe 12 en fonctionnement ; le circuit est alors vidangé. Le temps de rinçage, c'est-à-dire le temps compris entre la mise en route de la pompe 11 et l'arrêt des pompes 2 et 4 est fonction croissante de la valeur de ΔI retenue comme valeur de mesure. Sur la figure 2, T₁ et T'₁ désignent la durée de fonctionnement de la pompe 11, T₂ la durée de fonctionnement des pompes 2 et 4 et T₃ la durée de fonctionnement de la pompe 12. Si on désire mesurer la concentration en un autre produit, on peut effectuer après un premier cycle de mesure I, un second cycle de mesure II qui se déroule de façon analogue. Dans ce cas, les mesures sont effectuées successivement.

Si on désire effectuer plusieurs mesures simultanément, les différentes phases du processus de mesure sont déclenchées par l'ordinateur pour les temps T'₁, T₂, T₃ correspondant à la réponse enzymatique la plus élevée. Dans le cas illustré sur la figure 2, les différentes phases du procédé seraient déclenchées par les temps correspondant au produit dosé dans le cycle II.

## Revendications

1. Dispositif permettant le dosage direct sur la peau d'au moins un composé, comportant une cellule de mise en solution du composé à doser à l'aide d'un liquide de dissolution circulant sur la peau dans la cellule et au moins une électrode enzymatique, caractérisé par le fait qu'il comprend :
- un réservoir (1) pour le liquide de dissolution,
- une cellule (3) de mise en solution alimentée par le réservoir (1),
- au moins une électrode enzymatique (6), dont la membrane est en contact avec le liquide de dissolution provenant de la cellule (3) de mise en solution, et
- au moins une pompe (2) permettant de faire circuler le liquide de dissolution, de la cellule (3) vers l' (les) électrode(s) enzymatique(s) (6a, 7a, 7b, 7c), avec un retour (1a) au réservoir (1).

2. Dispositif selon la revendication 1, caractérisé par le fait que l'on utilise pour assurer la circulation du liquide de dissolution deux pompes (2, 4), la première (2) étant disposée en amont de la cellule (3) de mise en solution et la seconde (4) étant disposée en aval de la cellule (3).

3. Dispositif selon la revendication 2, caractérisé par le fait que la capacité d'aspiration de la pompe (4) située en aval de la cellule (3) est supérieure à la capacité de refoulement de la pompe (2) située en amont de ladite cellule (3).

4. Dispositif selon l'une des revendications 1 à 3, caractérisé par le fait qu'il comprend des pompes (11, 12) disposées sur la conduite d'alimentation du réservoir et sur la conduite de vidange du réservoir.

5. Dispositif selon l'une des revendications 1 à 4, caractérisé par le fait que la (les) pompe(s) est (sont) péristaltique(s).

6. Dispositif selon l'une des revendications 1 à 5, caractérisé par le fait qu'il est associé à une unité (14, 15) d'acquisition des informations fournies par l' (ou les) électrode(s) et de commande des pompes de circulation du liquide de dissolution.

7. Dispositif selon la revendication 4, caractérisé par le fait qu'une cuve (1O) de stockage est reliée au réservoir (1) par l'intermédiaire d'une pompe (11).

8. Dispositif selon l'une des revendications 1 à 7, caractérisé par le fait que le liquide de dissolution est une solution tampon.

9. Dispositif selon l'une des revendications 1 à 8, caractérisé par le fait que le réservoir (1) est thermostaté.

10. Dispositif selon l'une des revendications 1 à 9, caractérisé par le fait que la cellule (3) de mise en solution est constituée par une plaquette (2O2) de matière plastique sur une surface de laquelle est ménagé un compartiment (2O3) ouvert.

11. Dispositif selon la revendication 1O, caractérisé par le fait que le compartiment (2O3) a la forme d'une conduite enroulée en spirale.

12. Dispositif selon l'une des revendications 1 à 9, caractérisé par le fait que la (les) électrode(s) enzymatique(s) (6a, 7a, 7b) de mesure est (sont) associée(s) à au moins une électrode de référence (6b, 7c) qui a la même structure que la (les) électrode(s) enzymatique(s), à laquelle (aux-quelles) elle(s) est (sont) associée(s), à l'exception du fait que la membrane ne contient pas d'enzyme.

13. Dispositif selon l'une des revendications 1 à 12, caractérisé par le fait que les électrodes sont fixées sur des supports (8, 9).

14. Dispositif selon la revendication 13, caractérisé par le fait que les électrodes (6a, 6b ; 7b, 7c) sont fixées sur un même support (8, 7) sont des électrodes enzymatiques du même type et l'électrode de référence correspondante.

15. Dispositif selon l'une des revendications 13 ou 14, caractérisé par le fait que le réservoir (5O1) et le support d'électrodes sont combinés, le réservoir (5O1) comportant des éléments (512a, 512b, 512c, 512d) de support pour les électrodes.

16. Procédé de dosage direct sur la peau d'au moins un composé, utilisant le dispositif selon l'une des revendications 1 à 15, caractérisé par les opérations suivantes :
- on remplit le réservoir (1) avec le liquide de dissolution ;
- on applique la cellule (3) de mise en solution du composé à doser sur la zone de peau choisie ;
- on met en fonctionnement la (les) pompe(s) de circulation (2, 4) du liquide de dissolution pendant la durée déterminée et on met sous tension constante l' (ou les) électrode(s) enzymatique(s) et, éventuellement, de référence ;
- on maintient la circulation du liquide de dissolution et, simultanément, on repère l'intensité à la sortie de l' (les) électrode(s) enzymatique(s) (6a, 7a, 7b) et, éventuellement, de référence (6b, 7c), pour définir la quantité du (ou des) composé(s) dosé(s) ;
- on rince le circuit de circulation à l'aide du liquide de dissolution pur ; et
- on vidange ledit circuit.

17. Procédé selon la revendication 16, caractérisé par le fait que la valeur du signal fourni par une électrode est prise en compte comme valeur de mesure lorsque sa variation en fonction du temps devient inférieure à un seuil prédéfini.

18. Procédé selon la revendication 16, caractérisé par le fait que la pente d'évolution du signal fourni par une électrode est prise en compte comme valeur de mesure.

19. Procédé selon l'une des revendications 16 à 18, caractérisé par le fait que le temps de rinçage est fonction de la concentration du composé dosé dans le liquide de dissolution à la fin de la phase de mesure.

## Claims

1. Device making possible the direct quantitative determination on the skin of at least one compound, containing a cell for dissolving the compound to be quantitatively determined using a solubilizing liquid circulating over the skin in the cell and at least one enzymatic electrode, characterized in that it comprises:
- a reservoir (1) for the solublizing liquid,
- a solubilizing cell (3) supplied by the reservoir (1),
- at least one enzymatic electrode (6), the membrane of which is in contact with the solubilizing liquid arising from the solubilizing cell (3), and
- at least one pump (2) making it possible to circulate the solubilizing liquid from the cell (3) towards the enzymatic electrode(s) (6a, 7a, 7b, 7c), with a return (1a) to the reservoir (1).

2. Device according to Claim 1, characterized in that two pumps (2, 4) are used to provide for circulation of the solubilizing liquid, the first (2) being arranged upstream of the solubilizing cell (3) and the second (4) being arranged downstream of the cell (3).

3. Device according to Claim 2, characterized in that the suction capacity of the pump (4) situated downstream of the cell (3) is greater than the pump capacity of the pump (2) situated upstream of the said cell (3).

4. Device according to one of Claims 1 to 3, characterized in that it comprises pumps (11, 12) arranged on the supply pipe of the reservoir and on the discharge pipe of the reservoir.

5. Device according to one of Claims 1 to 4, characterized in that the pump(s) is (are) peristaltic.

6. Device according to one of Claims 1 to 5, characterized in that it is combined with a unit (14, 15) for acquisition of data supplied by the electrode(s) and for controlling the pumps for circulating the solubilizing liquid.

7. Device according to Claim 4, characterized in that a storage vessel (10) is connected to the reservoir (1) via a pump (11).

8. Device according to one of Claims 1 to 7, characterized in that the solubilizing liquid is a buffer solution.

9. Device according to one of Claims 1 to 8, characterized in that the reservoir (1) is thermostatically controlled.

10. Device according to one of Claims 1 to 9, characterized in that the solubilizing cell (3) consists of a plastic plate (202) provided on one of its surfaces with an open compartment (203).

11. Device according to Claim 10, characterized in that the compartment (203) has the form of a pipe wound into a spiral.

12. Device according to one of Claims 1 to 9, characterized in that the enzymatic measuring electrode(s) (6a, 7a, 7b) is (are) combined with at least one reference electrode (6b, 7c) which has the same structure as the enzymatic electrode(s) with which it (they) is (are) combined, with the exception of the fact that the membrane does not contain an enzyme.

13. Device according to one of Claims 1 to 12, characterized in that the electrodes are fixed to supports (8, 9).

14. Device according to Claim 13, characterized in that the electrodes (6a, 6b; 7b, 7c) are fixed to one and the same support (8, 7) and are enzymatic electrodes of the same type and the corresponding reference electrode.

15. Device according to either of Claims 13 and 14, characterized in that the reservoir (501) and the electrode support are combined, the reservoir (501) containing units (512a, 512b, 512c, 512d) for supporting the electrodes.

16. Process for the direct quantitative determination on the skin of at least one compound using the device according to one of Claims 1 to 15, characterized by the following operations:
- the reservoir (1) is filled with the solubilizing liquid;
- a cell (3) for dissolving the compound to be quantitatively determined is applied to the chosen skin region;
- the pump(s) (2, 4) for circulating the solubilizing liquid are operated for the specified period of time and the enzymatic and, optionally, reference electrode(s) is (are) placed under a constant voltage;
- the circulation of the solubilizing liquid is maintained and, simultaneously, the output current of the enzymatic (6a, 7a, 7b) and, optionally, reference (6b, 7c) electrode(s) is registered, in order to define the amount of the compound(s) quantitatively determined;
- the circulation circuit is rinsed using pure solubilizing liquid; and
- the said circuit is emptied.

17. Process according to Claim 16, characterized in that the value of the signal supplied by an electrode is taken into account as the measurement value when its variation as a function of time becomes less than a predefined threshold.

18. Process according to Claim 16, characterized in that the slope of the change in the signal supplied by an electrode is taken into account as the measurement value.

19. Process according to one of Claims 16 to 18, characterized in that the rinsing time is a function of the concentration of the compound quantitatively determined in the solubilizing liquid at the end of the measurement stage.

## Patentansprüche

1. Vorrichtung zur direkten Bestimmung wenigstens einer chemischen Verbindung auf der Haut, welche eine Zelle zum in Lösung bringen der zu bestimmenden Verbindung mit Hilfe einer in der Zelle auf der Haut zirkulierenden Lösungsflüssigkeit und wenigstens eine enzymatische Elektrode aufweist, dadurch gekennzeichnet, daß sie umfaßt:
- einen Behälter (1) für die Lösungsflüssigkeit,
- eine Zelle (3) zum in Lösung bringen, die von dem Behälter (1) beschickt wird,
- wenigstens eine enzymatische Elektrode (6), deren Membran mit der von der Auflösungszelle (3) herkommenden Lösungsflüssigkeit in Kontakt steht und
- wenigstens eine Pumpe (2), welche die Lösungsflüssigkeit von der Zelle (3) in Richtung der enzymatischen Elektrode(n) (6a,7a,7b,7c) mit einem Rücklauf (1a) zum Behälter (1) zirkulieren läßt.

2. Vorrichtung gemäß Anspruch 1, dadurch gekennzeichnet, daß man zur Aufrechterhaltung der Zirkulation der Lösungsflüssigkeit zwei Pumpen (2,4) verwendet, wobei die erste (2) stromaufwärts von der Auflösungszelle (3) und die zweite (4) stromabwärts von der Zelle (3) angeordnet sind.

3. Vorrichtung gemäß Anspruch 2, dadurch gekennzeichnet, daß die Ansaugleistung der stromabwärts von der Zelle (3) angeordneten Pumpe (4) größer ist als die Förderleistung der stromaufwärts von der Zelle (3) angeordneten Pumpe (2).

4. Vorrichtung gemäß einem der Ansprüche 1 bis 3, dadurch gekennzeichnet, daß sie Pumpen (11,12) umfaßt, die in der Versorgungsleitung des Behälters und in der Ablaßleitung des Behälters angeordnet sind.

5. Vorrichtung gemäß einem der Ansprüche 1 bis 4, dadurch gekennzeichnet, daß die Pumpe(n) peristaltisch arbeitet (arbeiten).

6. Vorrichtung gemäß einem der Ansprüche 1 bis 5, dadurch gekennzeichnet, daß sie mit einer Einheit (14,15) zur Erfassung von Informationen, die von der (oder den)
Elektrode(n) geliefert wird (werden) und zur Steuerung der Zirkulationspumpen für die Lösungsflüssigkeit verbunden ist.

7. Vorrichtung gemäß Anspruch 4, dadurch gekennzeichnet, daß ein Vorratsbehälter (10) unter Zwischenschaltung einer Pumpe (11) mit dem Behälter (1) verbunden ist.

8. Vorrichtung gemäß einem der Ansprüche 1 bis 7, dadurch gekennzeichnet, daß die Lösungsflüssigkeit eine Pufferlösung ist.

9. Vorrichtung gemäß einem der Ansprüche 1 bis 8, dadurch gekennzeichnet, daß der Behälter (1) thermostatisiert ist.

10. Vorrichtung gemäß einem der Ansprüche 1 bis 9, dadurch gekennzeichnet, daß die Auflösungszelle (3) aus einer Kunststoffscheibe (202) besteht, in deren einer Oberfläche eine offene Kammer (203) ausgespart ist.

11. Vorrichtung gemäß Anspruch 10, dadurch gekennzeichnet, daß die Kammer (203) die Form einer spiralförmig aufgerollten Leitung aufweist.

12. Vorrichtung gemäß einem der Ansprüche 1 bis 9, dadurch gekennzeichnet, daß die enzymatische(n) Meßelektrode(n) (6a,7a,7b) mit wenigstens einer Referenzelektrode (6b,7c) verbunden ist (sind), die abgesehen von der Tatsache, daß die Membran kein Enzym enthält, den gleichen Aufbau aufweist (aufweisen), wie die enzymatische(n) Meßelektrode(n) mit der (denen) sie verbunden ist (sind.

13. Vorrichtung gemäß einem der Ansprüche 1 bis 12, dadurch gekennzeichnet, daß die Elektroden auf Trägern (8,9) befestigt sind.

14. Vorrichtung gemäß Anspruch 13, dadurch gekennzeichnet, daß die auf dem gleichen Träger (8,7) befestigten Elektroden (6a,6b;7b,7c) enzymatische Elektroden des gleichen Typs und die entsprechende Referenzelektrode sind.

15. Vorrichtung gemäß einem der Ansprüche 13 oder 14, dadurch gekennzeichnet, daß der Behälter (501) und der Elektrodenträger zusammengefaßt sind, wobei der Behälter (501) Trägerbauteile (512a,512b,512c.512d) für die Elektroden aufweist.

16. Verfahren zur direkten Bestimmung wenigstens einer chemischen Verbindung auf der Haut unter Verwendung der Vorrichtung gemäß einem der Ansprüche 1 bis 15, gekennzeichnet durch die folgenden Schritte:
- man füllt den Behälter (1) mit der Lösungsflüssigkeit;
- man legt die Zelle (3) zum in Lösung bringen der zu bestimmenden Verbindung auf den gewählten Hautbereich;
- man schaltet die Zirkulationspumpe(n) (2,4) für die Lösungsflüssigkeit für eine bestimmte Zeit ein und man legt eine konstante Spannung an die enzymatische(n) und gegebenenfalls an die Referenzelektrode(n);
- man hält die Zirkulation der Lösungsflüssigkeit aufrecht und mißt gleichzeitig die Intensität am Austritt der enzymatischen Elektrode(n) (6a,7a,7b) und gegebenenfalls der Referenzelektrode (6b,7c), um die Menge der zu bestimmenden Verbindung(en) festzustellen;
- man spült den Zirkulationskreislauf mit Hilfe der reinen Lösungsflüssigkeit; und
- man entleert den Kreislauf.

17. Verfahren gemäß Anspruch 16, dadurch gekennzeichnet, daß der Wert des von einer Elektrode gelieferten Signals als Meßwert verwendet wird, wenn seine Änderung als Funktion der Zeit geringer als eine bestimmte Schwelle wird.

18. Verfahren gemäß Anspruch 16, dadurch gekennzeichnet, daß die Steigung des von einer Elektrode gelieferten Signalverlaufs als Meßwert verwendet wird.

19. Verfahren gemäß einem der Ansprüche 16 bis 18, dadurch gekennzeichnet, daß die Spüldauer eine Funktion der Konzentration der analysierten Verbindung in der Lösungsflüssigkeit am Ende der Meßphase ist.
